**Europäisches Patentamt**

**European Patent Office**

**Office européen des brevets**

(19)

(11) Veröffentlichungsnummer : **0 418 600 B1**

(12) **EUROPÄISCHE PATENTSCHRIFT**

(45) Veröffentlichungstag der Patentschrift :
**22.07.92 Patentblatt 92/30**

(51) Int. Cl.⁵ : **A61K 31/52,** A61K 9/16,
A61K 9/52

(21) Anmeldenummer : **90116423.6**

(22) Anmeldetag : **28.08.90**

(54) **Verfahren zur Herstellung von Xanthinderivat-Pellets.**

(30) Priorität : **08.09.89 DE 3929864**

(43) Veröffentlichungstag der Anmeldung :
**27.03.91 Patentblatt 91/13**

(45) Bekanntmachung des Hinweises auf die
Patenterteilung :
**22.07.92 Patentblatt 92/30**

(84) Benannte Vertragsstaaten :
**AT BE CH DE DK ES FR GB GR IT LI NL SE**

(56) Entgegenhaltungen :
**EP-A- 0 270 305**
**WO-A-88/09167**
**FR-A- 2 221 178**

(73) Patentinhaber : **NORDMARK ARZNEIMITTEL**
**GmbH.**
**Pinnauallee 4**
**W-2082 Uetersen (DE)**

(72) Erfinder : **Moest, Thomas, Dr.**
**An der Duene 9**
**W-2082 Moorrege (DE)**
Erfinder : **Pich, Claus Hinrich, Dr.**
**An der Duene 3**
**W-2082 Moorrege (DE)**

(74) Vertreter : **Schweiss, Werner, Dr. et al**
**BASF Aktiengesellschaft Patentabteilung**
**Carl-Bosch-Strasse 38**
**W-6700 Ludwigshafen (DE)**

## Beschreibung

Die Erfindung betrifft ein einfaches Verfahren zur Herstellung von Pellets mit ausgeprägt kugelförmiger Struktur, die zu 100 % aus Xanthin-Derivaten bestehen, deren Retardierung und daraus hergestellte Arzneimittel.

Zum Aufbau von Pellets nach herkömmlichen Technologien werden Nonpareilles als Starterkerne zum Aufdragieren oder Klebemittel und/oder andere Hilfsstoffe zur Herstellung einer plastisch verformbaren Masse benötigt.

Diese Herstellverfahren sind aufwendig, und sie erlauben nicht die Herstellung von Xanthinderivatpellets mit maximaler - nämlich 100 % - Wirkstoffkonzentration, die Wirkstoffdichte ist daher begrenzt. Eine hohe Wirkstoffdichte ist jedoch erforderlich, um höhere Arzneistoffmengen in Hartgelatine-Kapseln akzeptabler Größe abfüllen zu können.

Reine Theophyllinagglomerate sind ebenfalls bekannt, man kann sie entweder durch Abbaugranulation von kompaktiertem Theophyllin erhalten oder gem. DE-OS 37 12 058 durch das Pressen von Theophyllin/Wasser-Mischungen durch Lochscheiben zu Strangpreßlingen. Diese Formlinge haben, wie der Fachmann weiß, nicht die wünschenswerte Kugelform zur Erzielung einer guten Fließfähigkeit und einer maximalen Schüttdichte. In DE-OS 37 12 058 ist zwar die Weiterverarbeitung zu runden Pellets erwähnt, doch wäre hierzu ein zusätzlicher Verfahrensschritt notwendig, so daß drei Verfahrensstufen zur Formgebung resultieren würden: Mischen - Extrudieren - Ausformen. Abgesehen davon ist unklar, wie dieses Ausformen zu runden Pellets erfolgen soll, da die feuchte Theophyllinmasse gern. den Ausführungen in DE-OS 37 12 058 sehr schnell zum Erstarren neigt.

Der Erfindung lag die Aufgabe zugrunde, auf einfache Weise möglichst präzis kugelförmige Pellets aus Xanthin-Derivaten mit 100 % Wirkstoffkonzentration herzustellen, die zugleich eine hohe Schüttdichte von mehr als 0,5 g/cm$^3$ aufweisen und somit eine hohe Wirkstoffdichte, z.B. in Kapseln, ermöglichen.

Die Lösung dieser Aufgabe besteht in einem Verfahren zur Herstellung von aus Xanthin-Derivaten bestehenden Pellets mit ausgeprägt kugeliger Struktur, einer Korngröße im Bereich von 0,3 bis 4 mm und einer Schüttdichte über 0,5 durch Umsetzen von Xanthin-Derivaten mit Wasser oder Wasser/Alkohol-Mischungen, wobei man Xanthinderivat-Pulver einer mittleren Korngröße von 20 bis 200 μm unter Rühren in einer nicht lösenden, mit Wasser oder Wasser/Alkohol-Mischungen nicht mischbaren Flüssigkeit mit einem Siedepunkt im Bereich von 60 bis 160°C bei 40 bis 70°C suspendiert, 10 bis 40 Gew.-%, bezogen auf das eingesetzte Xanthin-Derivat, Wasser oder Wasser/Alkohol-Mischungen zusetzt, dann um 5 bis 20 K pro Stunde auf Raumtemperatur abkühlen läßt, wobei die Rührgeschwindigkeit nach der Agglomeration auf etwa 1/3 bis 2/3 der ursprünglichen reduziert werden kann, die Pellets von der Flüssigkeit trennt und trocknet.

Xanthin-Derivate im Sinne dieser Erfindung sind 2- und 3-fach N-substituierte 2,6-Dihydroxypurine, vorzugsweise Coffein, Pentoxifyllin, Diprophyllin, Etofyllin, Doxofyllin und vor allem Theophyllin.

Die Wasser/Alkohol-Mischungen enthalten höchstens 50, vorzugsweise 20 oder weniger Prozent Alkohol.

Man dispergiert das Pulver in der Flüssigkeit unter stetigem Rühren im beginnenden turbulenten Bereich, wobei es gleichgültig ist, ob man das Pulver vorlegt und die Flüssigkeit dazugießt oder umgekehrt. Emulgatoren sind nicht erforderlich. Die Flüssigkeit kann von vornherein auf 40 bis 60, vorzugsweise 40 bis 50°C aufgeheizt sein oder nach der Suspendierung des Pulvers aufgeheizt werden. Nach Zusatz von Wasser oder Wasser/Alkohol-Mischung, gleichgültig ob langsam oder rasch, tropfenweise oder in einem Guß, läßt man die Temperatur pro Stunde um 5 bis 20, vorzugsweise etwa 10 K absinken, wobei die Rührgeschwindigkeit auf etwa 2/3 bis 1/3 reduziert werden kann, wenn die zunächst trübe Dispersion des Pulvers sich durch Agglomeration geklärt hat. Je nach Korngröße des vorgelegten Xanthinderivat-Pulvers, der Menge an zugegebenem Wasser oder Wasser/Alkohol-Mischung und der Rührerdrehzahl kann die mittlere Korngröße der Pellets im Bereich von 0,3 bis 4, vorzugsweise 0,8 bis 2 mm eingestellt werden. Die Korngrößenverteilung ist innerhalb dieses Bereiches in der Regel eng, die Teilchen sind gleichmäßig kugelförmig. Sie werden nach dem Abkühlen auf Raumtemperatur von der Flüssigkeit getrennt und in üblicher Weise (vgl. Lehrbücher der Pharmazeutischen Technologie) bei 60 bis 80°C getrocknet. Ihre Schüttdichte beträgt mehr als 0,5, vorzugsweise mehr als 0,6 g/cm$^3$. Sie sind in hohem Maße druck- und abriebfest. In einem Gerät zur Bestimmung der mechanischen Abriebfestigkeit (Born Friabimat SA 400, Fa. Born Gerätebau, D 3554 Gladenbach) wurde nach der sehr hohen Schüttelbelastung von 999 min$^{-1}$ und 180 sec Dauer nur ein Abrieb von max. 0,8 % ermittelt.

Die Mengenverhältnisse Xanthinderivat : nicht lösender, mit Wasser oder Wasser/Alkohol-Mischungen nicht mischbarer Flüssigkeit : Wasser oder Wasser/Alkohol-Mischung liegen im Bereich von 1 kg : 1 bis 4 l : 0,2 bis 0,4 l.

Als nicht lösende, mit Wasser oder Wasser/Alkohol-Mischungen nicht mischbare, im Bereich von 60 bis 160, vorzugsweise 70 bis 130°C siedende Flüssigkeit kommen vor allem mittelkettige Alkane wie Cyclohexan, Benzin oder Petrolether in Betracht. Mit "nichtlösend, mit Wasser oder Wasser/Alkohol-Mischungen nicht mischbar" ist gemeint, daß sich keine praktisch relevanten Mengen Xanthinderivat in Wasser oder Wasser/Alkohol-Mischungen lösen, mit anderen Worten, daß sich beim Mischen von je 2 der 3 Komponenten in den oben angegebenen Mengenverhältnissen stets zwei Phasen bilden.

Mit der "mittleren Korngröße" ist das Gewichtsmittel gemeint.

Mit "ausgeprägt kugeliger Struktur" der Pellets ist gemeint, daß die Durchmesser eines Kügelchens im rechten Winkel zueinander um maximal 25, vorzugsweise maximal 15 % voneinander abweichen.

Die Pellets können direkt oder nach dem überziehen mit einem retardierenden (und/oder magensaftresistenten) Lack in Steckkapseln oder in Dosierbeutel abgefüllt werden. Retardierende Lacke enthalten die für diesen Zweck üblichen, in wäßrigem Medium unabhängig vom pH-Wert unlöslichen, diffusionskontrollierenden Polymeren, wie sie in den galenischen Lehr- und Handbüchern beschrieben sind, beispielsweise Poly(meth)acrylate, Zellulosederivate und Vinylpolymere. Bedingt durch die hohe mechanische Festigkeit, die ausgeprägt kugelförmige Gestalt und die gleichmäßige Oberfläche der Pellets ist es möglich, eine kontrollierte Wirkstoff-Freisetzung mit sehr geringen Lackmengen in beliebigen Coatinggeräten auf einfache Weise zu erhalten. Die gleichmäßig kugelförmige Struktur der Pellets ermöglicht ein präzises Füllen von Kapseln in dichtester Kugelpackung und daher zu Kapseln mit hohem Wirkstoffgehalt.

Arzneimittel im Sinne der Erfindung, die die erfindungsgemäß hergestellten Pellets enthalten, sind Steckkapseln, Dosierbeutel sowie Tabletten, die aus den Pellets unter Verwendung von Bindemitteln und ggf. anderen üblichen galenischen Hilfsstoffen gepreßt werden.

Beispiel 1

In einem 3 l-Doppelmantelgefäß mit Propellerrührer und Stromstörern wurden 2 l Petrolether mit dem Siedebereich 100 bis 130°C auf 45°C unter Rühren erwärmt. Nach Zugabe von 1 kg Theophyllinpulver der mittleren Korngröße 100 μm wurde mit einer Umfangsgeschwindigkeit von 5,4 m/s suspendiert. 0,3 l Wasser wurden in die Suspension eingerührt. Nach Reduzieren der Umfangsgeschwindigkeit auf 3,4 m/s nach der Agglomeration der suspendierten Pulverteilchen und langsamem Abkühlen mit ca. 10 -K/h auf ca. 20°C wurden die entstandenen Pellets mit einer Nutsche separiert. Getrocknet wurde in einem geeigneten explosionsgeschützten Trockenschrank bei ca. 60°C.

Die Pellets wiesen ein Korngrößenband von 500 bis 1800 μm auf, ein ausgeprägtes Maximum lag bei 900 μm.

Die Schüttdichte nach DIN 53 468 betrug 0,62 g/cm³. Der Abrieb nach 15 min trockenem Wirbeln in einem Wirbelschichtgerät betrug weniger als 0,2 %, bestimmt über ein 100 μm-Sieb.

Beispiel 2

In einem 1 l-Doppelmantelgefäß mit Schrägblattrührer und Stromstörer wurden 600 ml Petrolether mit dem Siedebereich 100 bis 130°C unter Rühren auf 60°C erwärmt. Nach Zugabe von 200 g Pentoxifyllin-Pulver mit einem Kornband unter 200 μm wurde mit einer Umfangsgeschwindigkeit von 1,7 m/s suspendiert.

30 ml einer Wasser/Ethanol-Mischung wurden in die Suspension eingerührt. Nach Abkühlung mit ca. 10 K/h auf 20°C wurden die entstandenen Pellets mit einer Nutsche separiert und in einem geeigneten explosionsgeschützten Trockenschrank bei ca. 60°C getrocknet.

Die Pellets wiesen ein Korngrößenband zwischen 400 und 1300 μm mit einem Maximum bei 800 μm auf. Die Schüttdichte nach DIN 53 468 betrug 0,64 g/cm³.

Der Abrieb nach 15 min trockenem Wirbeln in einem Wirbelschichtgerät betrug weniger als 0,3 %, bestimmt über ein 100 μm-Sieb.

## Patentansprüche

1. Verfahren zur Herstellung von aus Xanthin-Derivaten bestehenden Pellets mit ausgeprägt kugeliger Struktur, einer Korngröße im Bereich von 0,3 bis 4 mm und einer Schüttdichte über 0,5 durch Umsetzen von Xanthin-Derivaten mit Wasser oder Wasser/Alkohol-Mischungen, dadurch gekennzeichnet, daß man ein Xanthinderivat-Pulver einer mittleren Korngröße von 20 bis 200 μm unter Rühren in einer nicht lösenden, mit Wasser oder Wasser/Alkohol-Mischungen nicht mischbaren Flüssigkeit mit einem Siedepunkt im Bereich von 60 bis 160°C bei 40 bis 70°C suspendiert, 10 bis 40 Gew.-%, bezogen auf das Xanthin-Derivat, Wasser oder eine Wasser/Alkohol-Mischung zusetzt, dann um 5 bis 20 K pro Stunde auf Raumtemperatur abkühlen läßt, wobei man die Rührgeschwindigkeit nach der Agglomeration auf etwa 1/3 bis 2/3 der ursprünglichen reduzieren kann, dann die Pellets von der Flüssigkeit trennt und trocknet.

2. Verfahren zur Herstellung von Retardpellets, dadurch gekennzeichnet, daß man nach Anspruch 1 hergestellte Xanthinderivat-Pellets mit mindestens einem diffusionskontrollierenden, in wäßrigem Medium nichtlöslichen Polymeren überzieht.

3. Arzneimittel zur peroralen Anwendung, das als Wirkstoff 100 bis 600 mg pro Einheitsdosis von nach Anspruch 1 oder 2 hergestellten Xanthinderivat-Pellets oder -Retardpellets oder Mischungen von beiden enthält.

## Claims

1. A process for the manufacture of pellets which are composed of xanthine derivatives and have a markedly spherical structure, have a particle size in the range from 0.3 to 4 mm and have a bulk density above 0.5 g/cm³ by reacting xanthine derivatives with water or water/alcohol mixtures, which comprises a powdered xanthine derivative with an average particle size of from 20 to 200 μm being suspended by stirring at from 40 to 70°C in a nonsolvent which is immiscible with water or water/alcohol mixtures and has a boiling point in the range from 60 to 160°C, then adding from 10 to 40 % by weight, based on the xanthine derivative, of water or water/alcohol mixtures and subsequently allowing to cool to room temperature at from 5 to 20K per hour, it being possible to reduce the stirring speed after agglomeration to about 1/3 to 2/3 of the original, and the pellets being separated from the liquid and dried.

2. A process for the manufacture of delayed release pellets, which comprises coating pellets of xanthine derivative manufactured as claimed in claim 1 being coated with at least one diffusion-controlling polymer which is insoluble in aqueous medium.

3. A drug for oral use which contains as active compound from 100 to 600 mg per unit dose of pellets or delayed release pellets of xanthine derivative manufactured as claimed in claim 1 or 2, or mixtures of the two.

## Revendications

1. Procédé de préparation de pellets constitués par des dérivés de xanthine, de structure sphérique prononcée, d'une grosseur de grain comprise entre 0,3 et 4 mm et d'une densité apparente supérieure à 0,5, par réaction de dérivés de xanthine avec de l'eau ou des mélanges eau/alcool, caractérisé par le fait que dans un liquide non dissolvant, non miscible avec l'eau ou des mélanges eau/alcool, d'un point d'ébullition compris entre 60 et 160 degrés C, on met en suspension, entre 40 et 70 degrés C en brassant, une poudre d'un dérivé de xanthine d'une grosseur moyenne de grain de 20 à 200 μm, on ajoute 10 à 40 % en poids, rapportés au dérivé de xanthine, d'eau ou d'un mélange eau/alcool, on laisse refroidir ensuite de 5 à 20 K par heure jusqu'à la température ordinaire, la vitesse de l'agitateur, après l'agglomération, pouvant être réduite d'environ 1/3 à 2/3 de la vitesse initiale, puis on sépare les pellets du liquide et on les sèche.

2. Procédé de préparation de pellets à effet retard, caractérisé par le fait que l'on enrobe des pellets de dérivés de xanthine préparés selon la revendication 1 avec au moins un polymère insoluble dans des milieux aqueux, réglant la diffusion.

3. Médicament pour l'administration orale, contenant comme principe actif 100 à 600 mg, par dose unitaire, de dérivés de xanthine sous forme de pellets ou pellets à effet retard ou mélanges des deux.